# EUROPEAN PATENT SPECIFICATION

(11) **EP 2 385 130 B1**
(45) Date of publication and mention of the grant of the patent: **19.03.2014**
(21) Application number: 10305473.0
(22) Date of filing: 03.05.2010
(51) Int. Cl.: C12N 15/82, C12P 21/02, C12N 5/04

(54) **Method for producing recombinant proteins from plant hairy roots**
Verfahren zur Herstellung rekombinanter Proteine aus haarigen Pflanzenwurzeln
Procédé de production de protéines recombinantes à partir de racines de plantes transformées

(43) Date of publication of application: 09.11.2011
(73) Proprietor: Université de Picardie Jules Verne, 80025 Amiens (FR)
(72) Inventor: Boitel-Conti, Michèle, 80000 Amiens (FR); Huet, Yoann, 80000 Amiens (FR); Guerineau, François, 80480 Salouël (FR); Ele Ekouna, Jean-Pierre, 80330 Longueau (FR)
(74) Representative: Cabinet Plasseraud

(56) References cited:
- WO-A2-2008/005988
- ZANG YUN-XIANG ET AL: "Metabolic Engineering of Indole Glucosinolates in Chinese Cabbage Hairy Roots Expressing Arabidopsis CYP79B2, CYP79B3, and CYP83B1" BIOTECHNOLOGY AND BIOPROCESS ENGINEERING, vol. 14, no. 4, July 2009 (2009-07), pages 467-473, XP002598327 ISSN: 1226-8372
- BORISJUK NIKOLAI V ET AL: "Production of recombinant proteins in plant root exudates." NATURE BIOTECHNOLOGY, vol. 17, no. 5, May 1999 (1999-05), pages 466-469, XP002598290 ISSN: 1087-0156
- AGOSTINI ELIZABETH ET AL: "Production of peroxidases by hairy roots of Brassica napus" PLANT CELL TISSUE AND ORGAN CULTURE, vol. 47, no. 2, 1996, pages 177-182, XP002598288 ISSN: 0167-6857
- GAUME A ET AL: "Rhizosecretion of recombinant proteins from plant hairy roots." PLANT CELL REPORTS, vol. 21, no. 12, August 2003 (2003-08), pages 1188-1193, XP002598291 ISSN: 0721-7714
- WOODS RYAN R ET AL: "Hairy-root organ cultures for the production of human acetylcholinesterase" BMC BIOTECHNOLOGY, vol. 8, December 2008 (2008-12), XP002598292 ISSN: 1472-6750

## Description

### FIELD OF THE INVENTION

The invention relates to a method for secreting recombinant proteins from transgenic hairy roots, in particular transgenic hairy roots obtained from plants belonging to the Brassicaceae family.

### BACKGROUND OF THE INVENTION

Proteins are amino-acid biopolymers synthesized by all living organisms. There are involved in virtually all aspects of the cell life. Enzymes drive and regulate the metabolism, structural proteins shape the cell, receptors and signaling proteins integrate environmental changes. Nowadays, proteins are widely used not only in the industrial field (enzymes in washing powder, food additive, paper bleaching agents...) but also for medical applications (vaccines and allergens, hormones, antibodies...). Prior to the development of molecular biology and recombinant DNA technology tools, the sole source of proteins of interest remained the producing organism itself. Indeed, insulin was formerly purified from swine while human growth hormone was extracted from human corpse tissues. Major drawbacks of these approaches were the limited availability of the starting material and the rather low content of the protein of interest. Moreover, the risk of viral contamination concerning proteins with medical applications remained high, especially when they were extracted from human tissues. In the 80', the recombinant DNA technology provided alternatives to such problems by allowing the overproduction of foreign proteins (recombinant proteins) in a given host organism. Indeed, animal insulin was the first recombinant protein with medical application to be produced in the bacterium *E.coli.* At the present time, cultures of animal cells and *E.coli* are the two references for the bioproduction of recombinant proteins.
However, bacteria are unable to produce complex (glycosylated) proteins and animal cell culture is a rather expensive process which cannot exclude the risk of animal virus contamination. Alternative bioproduction systems thus emerged during the last two decades, including plants which are considered to be safe (no viral risk), able to produce complex proteins and cheap to grow. Containment of plant-derived bioproduction systems (in greenhouses for whole plant, or bioreactors for plant cell and organ cultures) is preferred to plants grown in field. Hairy roots are an example of such confined bioproduction system as they can easily be cultured in bioreactors and transgenic clones can be obtained for any gene of interest. This particular root system is generated following infection of the plant cell by *Agrobacterium rhizogenes* which naturally transfer several bacterial genes (*rol* genes) into the plant genome. These *rol* genes force the infected plant cell (from stem, leaf...) to follow a new development program leading to the formation at the infection site of a new root system: the hairy roots. *A*. *rhizogenes* can be genetically modified in order to perform the transfer of a gene of interest (encoding a biopharmaceutical) for the production of transgenic hairy roots. Tobacco (*Nicotiana tabacum*) is by far the most largely used plant species for the production of recombinant protein but several others were shown to be suitable (Daniell et al., Trends in Plant Science, 2009). It is indeed a well characterized species which is largely used in academic and applied research. *Nicotiana tabacum* hairy roots were shown to be able to produce and secrete not only the model recombinant protein GFP but also several relevant proteins for therapeutic applications in human health.
For example, previous work by Medina-Bolivar (Medina-Bolivar *et al.,* 2004) showed the accumulation of eGFP in the medium of *Nicotiana tabacum* root culture at 0.8 mg/L after 21 days.
More recently, Woods *et al.* (Woods et al. 2008, BMC Biotechnology, 8:95) have produced human acetylcholinesterase from hairy-root organ cultures from *Nicotinia benthamiana.*

### SUMMARY OF THE INVENTION

The present invention relates to a method for obtaining a recombinant protein from hairy roots comprising the steps of:
a) transforming a plant with a strain of *Agrobacterium rhizogenes* and/or with a strain of *Agrobacterium tumefaciens* comprising the *rol* genes;
   and
b) transforming said plant with a vector containing an expression cassette comprising a signal peptide and a gene encoding said recombinant protein;
wherein said plant belongs to the Brassicaceae family.

Also described are hairy root cultures obtainable by the transformation steps described above.
The specification discloses a culture medium containing a recombinant protein obtainable by the method described above.

The specification further describes to a recombinant protein obtainable by the method described above.

Indeed, the inventors have discovered that hairy roots obtained by transforming a plant belonging to the Brassicaceae family with *Agrobacterium rhizogenes* are capable of secreting large amounts of recombinant protein into the extracellular medium. As an alternative, *Agrobacterium rhizogenes* can be replaced by *Agrobacterium tumefaciens,* in the presence of the *rol* genes, known to induce the formation of hairy roots.
One advantage of the present invention is to simplify the recovery and downstream processing of the recombinant protein. Another advantage is that the root biomass is not destroyed for protein recovery and a given culture can be used for several cycles of production of said recombinant protein. In contrast to plants grown in fields where environmental factors (temperature changes, drought, pest attacks, pesticide/herbicide use...) may considerably affect the production level of the recombinant protein, transgenic root culture conditions in bioreactors are controlled and standardized leading to a homogenous production among different batches. Moreover, roots do not produce pollen and can not survive outside the bioreactor, which eliminates the risk of transgene dissemination in the environment.

The use of edible plant species for several hundred years in human/animal nutrition is a good indication of their harmless nature. In contrast to tobacco, which has been used extensively in the past for this type of application, and which belongs to the Solanaceae family well known for the ability of several of its species member to produce potentially toxic compounds (alkaloids),including nicotine.
The use of an edible plant root system for the production of therapeutic recombinant proteins thus lowers safety concerns relative to the starting raw material prior to protein purification (no animal virus).
Thus, advantageously, the method of the invention enables the obtaining of high levels of recombinant proteins, with reduced purification/downstream processing costs, enables a new formulation for orally-delivered biopharmaceuticals and lowers safety concerns for human health and the environment

### DETAILLED DESCRIPTION OF THE INVENTION

In one aspect, the invention relates to a method for secreting a recombinant protein from hairy roots comprising the steps of:
a) transforming a plant with a strain of *Agrobacterium rhizogenes* and/or with a strain of *Agrobacterium tumefaciens* comprising the *rol* genes;
   and
b) transforming said plant with a vector containing an expression cassette comprising a signal peptide and a gene encoding said recombinant protein;
wherein said plant belongs to the Brassicaceae family.

In a preferred embodiment, a strain of *Agrobacterium rhizogenes* is used.
In another embodiment, a strain of *Agrobacterium tumefaciens* is used. In this embodiment, the strain of *Agrobacterium tumefaciens* is capable of inducing the formation of hairy roots if it harbors a pRi plasmid comprising the *rol* genes.

Typically, steps a) and b) are performed simultaneously.
In one embodiment, the strain of Agrobacterium itself contains an expression cassette comprising a gene encoding the recombinant protein.

In another embodiment, step b) is performed prior to step a)
In this embodiment, hairy roots can be obtained by transforming a transgenic plant which expressed the gene of interest with a strain of Agrobacterium under conditions which induce the formation of hairy roots.

Thus, in one embodiment the invention relates to a method as described above, wherein step a) and step b) are performed simultaneously by transforming said plant belonging to the Brassicaceae family with a strain of *Agrobacterium rhizogenes*, wherein said strain of *Agrobacterium rhizogenes* contains an expression cassette comprising a gene encoding said recombinant protein.

In other words, the invention relates to a method for obtaining a recombinant protein from hairy roots comprising the step of transforming a plant with a strain of *Agrobacterium rhizogenes,* wherein said plant belongs to the Brassicaceae family and wherein said strain of *Agrobacterium rhizogenes* contains an expression cassette comprising a signal peptide and a gene encoding said recombinant protein.

As used herein, the expression "plant belonging to the Brassicaceae family" has its general meaning in the art. It encompasses any plant of the Brassicaceae family, also known as the crucifers, the mustard family or cabbage family.
It contains over 330 genera and about 3,700 species, according to the Royal Botanic Gardens, Kew.
The largest genera are Draba (365 species), Cardamine (200 species, but its definition is controversial), Erysimum (225 species), Lepidium (230 species) and Alyssum (195 species.) Well-known species: Brassica oleracea (cabbage, cauliflower, etc.), Brassica rapa (turnip, Chinese cabbage, etc.), Brassica napus (rapeseed, etc.), Raphanus sativus (common radish), Armoracia rusticana (horseradish), Matthiola (stock), Arabidopsis thaliana (model organism) and many others. Among these species, several produce edible roots (turnip, radish...)

In a preferred embodiment, said plant belonging to the Brassicaceae family is selected from the group consisting of *Raphanus sativus, Raphanus sativus var. niger, Brassica Oleracea L. Convar, Brassica rapa and Arabidopsis thaliana.*
Even more preferably, said plant belonging to the Brassicaceae family is *Brassica rapa*

Transformation by *Agrobacterium rhizogenes* and/or *Agrobacterium tumefaciens* is a known technique in the art. The skilled person is familiar with the different techniques commonly employed for carrying out said transformation step. According to the species to be transformed, different parts of the plant can be used for the infection (hypocotyls, leaves etc.). Typically, infection by *Agrobacterium rhizogenes* and/or *Agrobacterium tumefaciens* is carried out by applying an *Agrobacterium rhizogenes* and/or *Agrobacterium tumefaciens* inoculum to plant tissues which has been previously wounded.

Several strains of *Agrobacterium rhizogenes* can be used for carrying out the invention. Suitable strains include, but are not limited to, the *Agrobacterium rhizogenes* strain TR7, also known as ATCC 25818 and the strains LBA 9402, A4T, A4, LBA1334, ATCC 11325, ATCC 15834 and LMG 155.
In a preferred embodiment, said strain of *Agrobacterium rhizogenes* is the strain ATCC 25818.

Several strains of *Agrobacterium tumefaciens* can be used for carrying out the invention. Suitable strains include, but are not limited to, *A*. *tumefaciens* C58, C58C1, LBA4404, GV2260, GV3100, A136, GV3101, GV3850, EHA101, EHA105, AGL-1.

The expression "*rol* genes" as used herein has its general meaning in the art. It refers to the group of bacterial genes which are capable of inducing the formation of hairy roots (Schmulling *et al.,* 1988; Bulgakov *et al.,* 2008 and references therein). Typically, the *rol* genes are harbored by a plasmid such as a pRi plasmid.

As used herein, the term "expression cassette" has its general meaning in the art. It refers to a nucleic acid construct, which, when present in a given cell under suitable condition, enables the expression of a gene of interest. According to the present invention, said gene of interest is gene encoding a recombinant protein which one wishes to produce and collect.
The expression cassette can be contained in any suitable expression vector. Typically, the expression may be a binary vector, such as the pRD400 binary vector, which has been modified to include the gene encoding the recombinant protein of interest.

In one embodiment, said expression cassette comprises a promoter, a signal peptide, a gene encoding said recombinant protein and a polyadenylation sequence.

In one embodiment, the promoter is a promoter derived from a Brassicaceae plant-infecting virus (Cauliflower Mosaic Virus).
In a preferred embodiment, said promoter is the CaMV35S promoter.
In one embodiment, said promoter is an heat or nutrient inducible promoter, such as Arabidopsis heat shock protein (Hsp) and transcription factor (Hsf) promoters (William R Swindell *et al.,* 2007) or the promoter of the At2g33830 Arabidopsis gene.

According to the present invention, the expression cassette comprises a signal peptide. In one embodiment, said signal peptide is the native signal peptide comprised in the gene encoding the recombinant protein which is to be expressed and secreted. In an alternative embodiment, said signal peptide is derived from a Brassicaceae plant. In a preferred embodiment, said signal peptide is a signal peptide from the Arabidopsis pectin methylesterase AT1G69940 or a variant thereof which differs by the replacement, deletion or addition of one or several amino acids, typically by the addition of 1, 2, 3 or 4 amino acids. In a preferred embodiment, said signal peptide is the signal peptide as set forth in SEQ ID NO:1.

The method of the invention is suitable for obtaining any type of recombinant protein. As used, the expressions "recombinant protein" or "heterologous protein" or "protein of interest" are used interchangeably to refer to a protein which is usually not expressed by the plant belonging to the Brassicaceae family, or not expressed at significant levels.
Suitable recombinant proteins according to the invention include, but are not limited to, allergens, vaccines, enzymes, enzyme inhibitors, antibodies, antibody fragments, antigens, toxins, anti-microbial peptides, hormones, growth factors, blood proteins (such as albumin, coagulation factors, transferrin), receptors and signaling proteins, protein component of biomedical standards, protein component of cell culture media, fusion or tagged proteins, cystein (disulfide bridges)-rich peptides and proteins, and glycosylated plant proteins (such as lectins, papain..).

In a preferred embodiment, said recombinant protein is selected from the group consisting of monoclonal antibodies and antibody fragments (single chain FV...), antigens especially from bacteria, virus or fungi sources, allergens, immunoregulators, digestive enzymes and orally-delivered proteins with therapeutic use (lipase, pepsine...).

Also described is a hairy root culture obtainable by:
a) transforming a plant with a strain of *Agrobacterium rhizogenes* and/or with a strain of *Agrobacterium tumefaciens* comprising the *rol* genes;
   and
b) transforming said plant with a vector containing an expression cassette comprising a gene encoding said recombinant protein;
wherein said plant belongs to the Brassicaceae family.

Also described is a hairy root culture obtainable by transforming a plant with a strain of *Agrobacterium rhizogenes,* wherein said plant belongs to the Brassicaceae family and wherein said strain of *Agrobacterium rhizogenes* contains an expression cassette comprising a gene encoding said recombinant protein, wherein said transformation is as defined above.

In one embodiment, the culture medium containing said recombinant is collected and is directly used for future applications.
Also described is a hairy root culture medium which contains a recombinant protein, obtainable by the method described above.
Advantageously, the culture medium containing the recombinant protein is suitable for oral use in humans and/or animals without any purification step.

In an alternative embodiment, the recombinant protein is obtained after one or several purification steps. Typically, the culture medium can first be clarified by standard filtration or low speed centrifugation in order to remove cell fragments. The protein of interest is then either precipitated by high salt concentration and dialyzed or directly fixed on an affinity chromatography column. The concentrated recombinant protein can then either be lyophilized or stored in an appropriate storage buffer at low temperature.
Suitable protocols adapted to each recombinant protein obtainable according to the invention and to each type of application envisaged are standard techniques in the art, and the skilled person will readily select the appropriate purification step(s), if any, for the desired application.

Also described is a recombinant protein obtainable by the method as defined above. Advantageously, said recombinant protein has a purity of at least 20%, preferably at least 25%, even more preferably 30%, 40%, 50%, 60%, 70%, 80%, 90%, 95%. In particular, it is devoid of animal proteins and animal pathogens (virus, prions...).

The invention will be further illustrated by the following examples. However, these examples should not be interpreted in any way as limiting the scope of the present invention.

### FIGURE LEGENDS

**Figure 1****:** schematic representation of the 6XHis-eGFP encoding pRD400 plasmid (pRD400-*gfp*).
**Figure 2****:** 6XHis-eGFP quantification in transgenic hairy root tissue of *Brassica rapa* (VER) and *Nicotiana tabacum* (Nt) species.
**Figure 3****:** intracellular eGFP content in the different parts of the cultured root biomass for the VER and the Nt species. The central part (zone 1) contains the oldest tissues while the peripheral part (zone 3) is composed by the young growing root tissues. The zone 2 is an intermediate part. Analysis was performed for root biomass cultured for 20 or 40 days.
**Figure 4****:** Secretion of 6XHis-eGFP over time in VER and Nt root cultures.
   **(A)** 26 µl of Ver or Nt root culture medium were sampled at the indicated times during the course of the culture and analysed 12.5% acrylamide gel. Total secreted proteins were revealed by a coomassie stain. 500 ng of bacterially expressed 6XHis-eGFP were loaded on the same gel as a quantitative and qualitative control. M: molecular marker.
   **(B)** Concentration of the secreted 6XHis-eGFP of the same samples as the panel A was determined by fluorescence measurements.

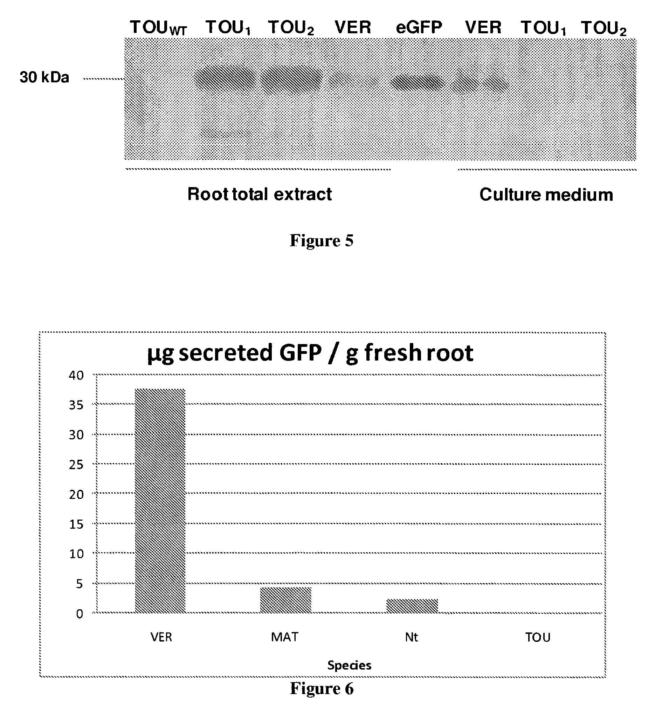

**Figure 5****: Western blot analysis of intra- and extra-cellular 6XHis-eGFP production for VER and TOU root culture.**
   Total soluble protein extract (intra- or extra-cellular) of TOU and VER 6XHis-eGFP-expressing hairy root clones were analyzed by western blot using a primary anti-GFP antibody. 50 µg of soluble proteins were loaded for each total root protein extract. For the detection of secreted 6XHis-eGFP, TCA precipitated proteins from 6 ml of the culture medium were loaded. TOU_{WT} is a wild type hairy root clone which was not transformed with the pRD400-gfp construct. TOU₁ and TOU₂ are two independent 6XHis-eGFP-expressing clones from the TOU species. TOU₁ is a 21 day-old root culture whereas all other clones were cultivated for 10 days. 100 ng of the commercially available pure 6XHis-eGFP were loaded as a quantitative and qualitative control.
**Figure 6****:** Performance for 6XHis-eGFP secretion for hairy root cultures of *Nicotiana tabacum* (Nt) and the three edible species *Brassica rapa* (Ver), *Spinacia oleracea* (MAT) and *Daucus carota* (TOU), as determined by fluorescence measurements.

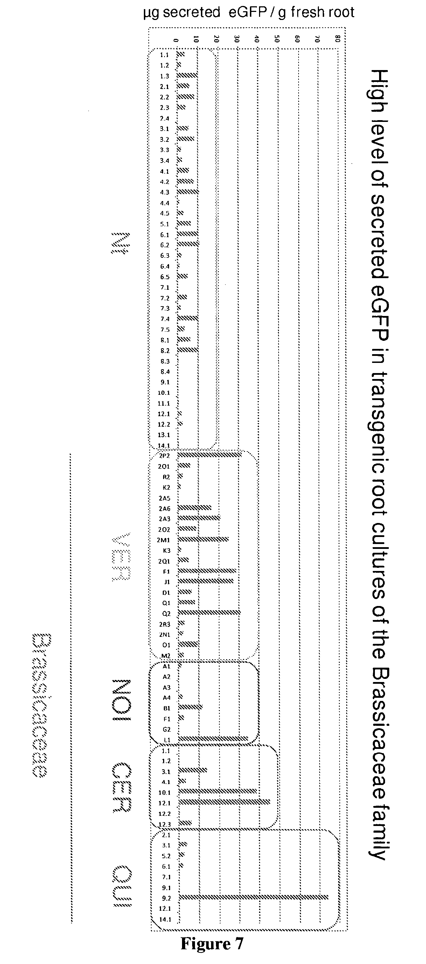

**Figure 7****:** Secreted 6XHis-eGFP was quantified by fluorometry in the culture medium of 20 day-old independent clones. The identification number of each independent clone is indicated on the abscise axis. *Brassica rapa* (VER), *Raphanus sativus var. niger* (NOI), *Raphatius sativus* (CER) and *Brassica Oleracea L. Convar* (QUI) belong to the Brassicaceae family, while *Nicotiana tabacum* (Nt) belongs to the Solanaceae family. Not represented on this graph are the *Daucus carota* (TOU) clones from the Apiaceae family which do not produce any detectable level of secreted 6XHis-eGFP.

### EXAMPLES

### I. Materiel and methods

### A. Recombinant binary plasmid for 6XHis-eGFP expression

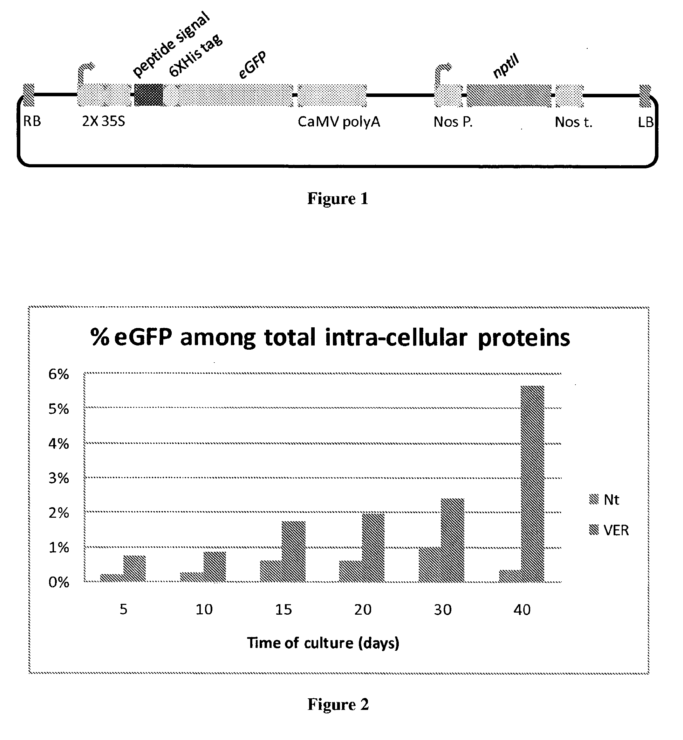

The coding sequence of 6XHis-eGFP (p6XHIS-EGFP, CLONTECH) was cloned in the Asp718 and BamHI restriction sites of the pRD400 plant expression vector (Datla et al., 1992), in frame with the 6XHis-tag and the secretion signal sequence from the Arabidopsis pectin methylesterase AT1G69940. In order to improve signal peptide cleavage, a C-terminal DP dipeptide was added to the naturally occurring signal peptide sequence to lead MGYTNVSILLGLLMVFVTPMVFADP (SEQ ID NO:1). Gene expression is driven by a duplicated CaMV35S promoter for 6XHis-eGFP and a Nos promoter for the nptII selectable marker (kanamycin resistance). The resulting plasmid, pRD400-gfp has the sequence set forth in SEQ ID NO:2.
A.rhizogenes strain ATCC 25818 was been transformed by electroporation with the empty or the 6XHis-eGFP encoding pRD400 plasmid (pRD400-gfp) (see Figure 1).

### B. Production, selection and culture of transgenic hairy roots

### 1. Plant species and culture in vitro

The following plant families and species (abbreviation in brackets) were used for hairy root production:

| **5 plant families** | **17 plant species (species name *abbreviation*)** |
|---|---|
| Apiaceae | *Daucus carota (TOU)* |
| Asteraceae | *Lactuca sativa (BRU)* |
| Brassicaceae | *Raphanus sativus (CER), Raphanus sativus var. niger (NOI), Brassica Oleracea L. Convar (QUI), Brassica rapa (VER)* |
| Chenopodiaceae | *Spinacia oleracea (MAT)* |
| Solanaceae | *Nicotiana tabacum (Nt)* |

Seeds were purchased from Gondian (www.gondian.com). They were surface sterilized with 70% ethanol for 5 min, 7 % bleach for 10 min and washed 5 times with sterile water before to be placed on half strength MS solid medium pH 5.8 supplemented with 1% sucrose. Germination and seedling growth occurred at 22°C with a 16h light/8h dark photoperiod.

### 2. Plant infection by Agrobacterium rhizogenes

We used the *Agrobacterium rhizogenes* strain TR7 (ATCC 25818) provided by the BCCM^{™}/LMG Bacteria Collection, Laboratorium Voor Microbiologie, Universiteit Gent. *Agrobacterium rhizogenes* was grown on MGL plates (2.5 g/l yeast extract, 5 g/l tryptone, 5 g/l mannitol, 5 g/l NaCl, 1.16 g/l Na-glutamate, 0.25 g/l KH2PO4, 0.1 g/l MgSO4, 1.0 mg /l biotin, 8 g /l Agar , pH 7.0) eventually supplemented with 50 mg /l kanamycin for selection of the binary plasmid. Inoculums were prepared from a 20 ml liquid bacterial culture grown overnight at 25°C in MGL medium. The suspension was centrifuged for 5 min at 15,000 rpm and collected cells were resuspended in fresh MGL medium and diluted to obtain an optical density of 1±0.1 at 600 nm.

Plant infection (except for *Nicotiana tabacum* and *Daucus carota*) was performed by pricking with a needle the hypocotyls of 3-10 day old seedlings and by applying the *Agrobacterium* inoculum with a sterile cotton swab on the injured zone. Depending on the plant species, hairy roots emerged from the wounded site 2 to 5 weeks after infection.

For *Nicotiana tabacum,* young leaves of 4-week old plant grown aseptically on half strength MS solid medium supplemented with 1% sucrose were cut at the petiole and transferred as a whole piece on half strength MS solid medium pH5.8 supplemented with 3% sucrose. The main central vein was then longitudinally sectioned, starting from the middle of the leaf toward the petiole extremity. *Agrobacterium* inoculum was then applied on the injured zone as described above. After 4 days, leaves were transferred on solid MS medium supplemented with 3% sucrose and 300 mg/l cefotaxime sodium (MS3cef medium) in order to get rid of the *Agrobacteria.* Numerous *Nicotiana tabacum* hairy roots emerged from de middle vein after about 4 weeks.
For *Daucus carota*, the first 2 leaves of 10-14 day old seedling were injured at the extremity of 3-4 leaflets using a forceps and immediately painted with a sterile cotton swab previously soaked in the *Agrobacterium* inoculum.

### 3. Selection and culture of 6XHis-eGFP-expressing hairy root clones

Infected hypocotyls developing hairy roots were cut out from the seedling and placed on MS3cef medium. After 7-10 days, 15 to 40 independent hairy root tips (except for MAT for which only 5 independent clones were available) were transferred on fresh MS3cef solid medium where they grow for 2-4 weeks. Root tips emerging from *Nicotiana tabacum* or *Daucus carota* leaves were treated in a similar manner. For each clone, a root fragment was directly checked for GFP expression by observing fluorescence emission using a Nikon Eclipse 90i microscope. Pictures were taken using a Nikon Digital Sight DS-5Mc camera. For each species, the 10 brightest clones were further cultured in liquid medium.
To initiate cultures in liquid medium, 5 pieces (about 1 cm long) of each hairy root clone were then transferred in a glass tube containing 5 ml of liquid B53cef medium consisting of B5 medium (Duchefa) pH 5.8 supplemented with 3% sucrose and 300 mg/l cefotaxime sodium and cultured for 10 days. Hairy roots were then successively cultured (each time for 3 weeks) in a 100 ml-Erlenmeyer containing 20 ml B53cef and in 250 ml-Erlenmeyer containing 100 ml of B53cef. After those steps aiming to get rid of the *Agrobacteria* by the use of the antibiotic cefotaxime, the standard culture conditions were the following: 100 ml B5 medium supplemented with 3% sucrose, pH 5.8 at 20-25°C under dim light, on a Gerhardt RO20 rotator (90 rpm/min). Root clones were sub-cultured every 3 weeks using 1g of root biomass for 100 ml of culture medium.

*Daucus carota* hairy roots were cultivated in MW medium (Bécard *et al.,* 1988) instead of the B5 medium.

### C. 6XHis-eGFP quantification by fluorometry

For secreted 6XHis-eGFP quantification, 50 µl of 1M Tris/HCl pH 8 was added to 500 µl aliquot of the root culture medium. The 6XHis-eGFP fluorescence was measured in a BioRad VersaFluo^{™} fluorometer (excitation filter: 485-495 nm; emission filter: 505-515 nm). Fluorometer calibration was performed using a commercially available recombinant 6XHis-eGFP (BioVision) used at a concentration of 1 to 10 mg/L.
For intracellular 6XHis-eGFP quantification, root tissue was ground in liquid nitrogen and an aliquot of the obtained tissue powder (equivalent to a spatula tip) was homogenized in 500 µl of ice cold 10 mM Tris/HCl pH8, and centrifuged at 4°C for 5 min at 14000g. Total protein quantification of the supernatant was performed using the Bradford method and the sample was diluted with 10 mM Tris/HCl pH8 until to reach a total protein concentration of 50 mg/L. The diluted sample was directly used for fluorescence measurements. The standard curve was performed using a commercially available recombinant 6XHis-eGFP (BioVision) diluted in wild-type hairy root extract with a total protein concentration of 50 mg/L.

### D. SDS-PAGE and WESTERN BLOT

Aliquots of hairy root culture medium were sampled at the indicated times, mixed with 1/3 volume of 3X Laemmli buffer and boiled for 5min. 40 µl of each sample were loaded on 12.5% acrylamide SDS-PAGE and the gel was stained with cooomassie blue. Commercially available 6XHis-eGFP expressed in E.coli (Biovision) was used as a quantitative and qualitative control.
Western blot on nitrocellulose membrane (Protean) were performed using the BioRad transfer system according to the manufacturer recommendations.

### II. Results

### A. Production level of intra-cellular 6XHis-eGFP in Nicotiana tabacum (Nt) and in the two edible Brassica rapa (VER) and Daucus carota (TOU) species

### 1. Microscopic observation of 6XHis-eGFP fluorescence

Microscopic observation of pRD400-gfp transformed plants, and non-transformed control plants, under white or blue light was carried out. Blue light allowed the observation of the 6XHis-eGFP green fluorescence in all three transformed species.

### 2. Intra-cellular 6XHis-eGFP quantification by fluorometry

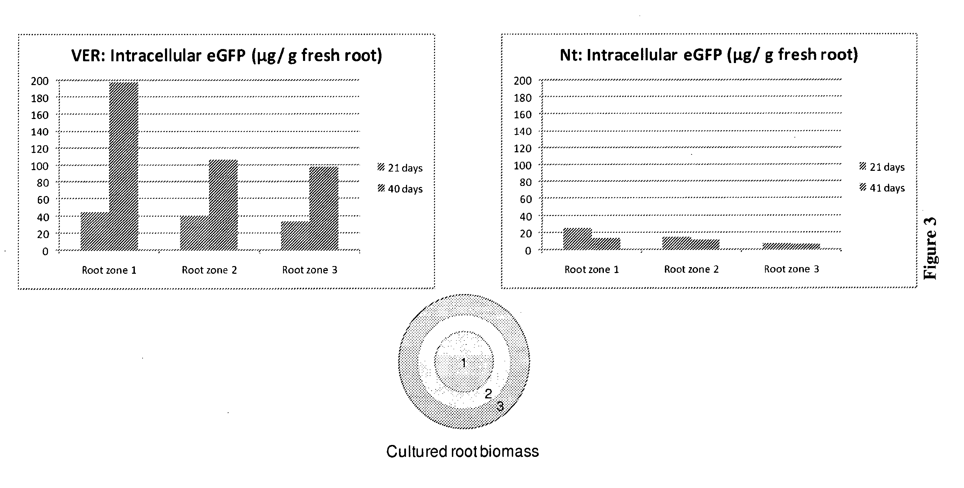

VER and Nt transgenic hairy root clones showed a similar rate production of intra-cellular 6XHis-eGFP during the growth phase (- the first 30 days). After - 40 days of culture, Nt hairy roots show a brownish color characteristic of dying tissues and stop to produce the recombinant protein. In contrast, VER roots show a white to slight yellowish color and not only continue to produce 6XHis-eGFP but also increase their production rate (Figure 2). Thus, root aging correlates with the increase in 6XHis-eGFP production for the VER species. This was confirmed by the analysis of the different parts of the cultured root biomass (Figure 3) which is composed by the central zone (zone 1: oldest root tissue), an intermediate one (zone 2), and the peripheral part consisting of very young and proliferating root tissues (zone 3).

### B. 6XHis-eGFP is efficiently secreted by the Brassica rapa (VER) species

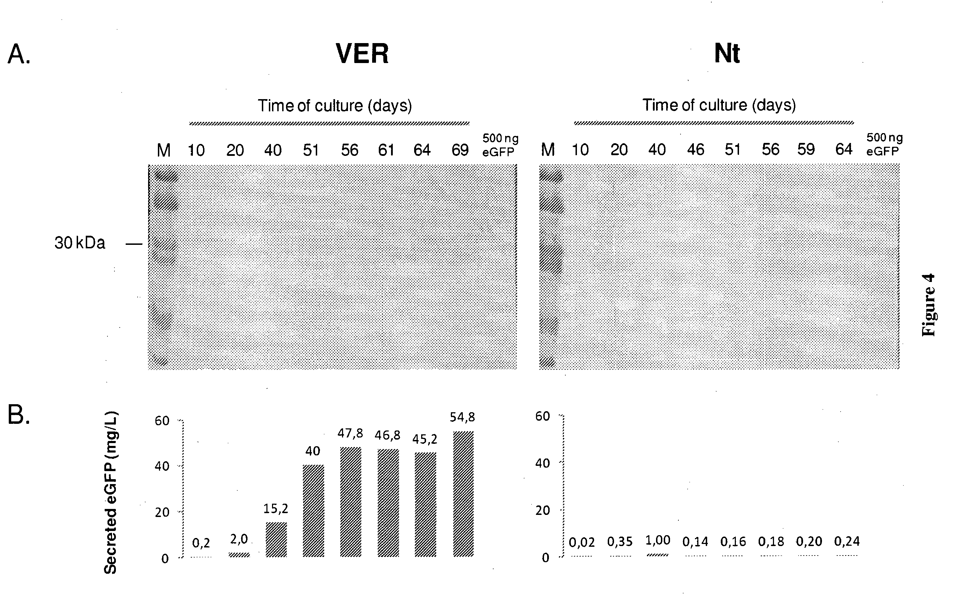

Accumulation of secreted 6XHis-eGFP in the culture medium of *Brassica rapa* (VER) and *Nicotiana tabacum* (Nt) hairy root cultures was monitored over time by SDS-PAGE and coomassie staining. The high level of 6XHis-eGFP secretion by the VER species allows the direct observation of the recombinant protein on the coomassie-stained acrylamide gel as shown in Figure 4 (panel A). While secreted 6XHis-eGFP is detected in the medium of the VER root culture right from the 10^{th} day, the recombinant protein is undetectable in the Nt root culture medium.
Fluorescence emission of the sampled culture media (Figure 4, panel B) correlates with the presence of the 26 kDa 6XHis-eGFP protein detected on the coomassie-stained gel and confirms its accumulation for the VER root culture over the first 51^{st} days, reaching a concentration of 40 mg/L. Both SDS-PAGE and fluorometric analysis revealed the remarkable stability of 6XHis-eGFP in the medium of the VER root culture. In contrast, 6XHis-eGFP concentration in the Nt culture medium raised at a maximum of 1 mg/L after 40 days, and decreased dramatically thereafter. This result is in accordance with previous work (Medina-Bolivar *et al.,* 2004) which showed the accumulation of eGFP in the medium of Nt root culture at 0.8 mg/L after 21 days.

### C. Brassica rapa (VER) is ahead of several other plant species for its capacity to secrete 6XHis-eGFP

In order to determine if the accumulation at high level of 6XHis-eGFP is a specificity of VER, transgenic hairy roots expressing 6XHis-eGFP were generated for several other plant species. We first analyzed by western blot the production and secretion of 6XHis-eGFP by TOU root cultures in comparison to the previously characterized VER clone (Figure 5).

The results show that although the TOU species produced similar amounts of intra-tissue 6XHis-eGFP, the recombinant protein was undetectable in the culture medium in contrast to the VER root culture. 6XHis-eGFP could be detected for both plant species in total intra-cellular soluble protein extract as shown by the revelation of a major band at about 30 kDa, the expected size for 6XHis-eGFP. Moreover, the bacterially expressed 6XHis-eGFP migrates at the same position. A minor band which likely represents a degradation product was also revealed in these total extracts. The presence of secreted 6XHis-eGFP could be clearly detected in the root culture medium of transgenic VER after 10 days of culture but not for the TOU root cultures at either 10 or 21 days of culture, indicating that although efficiently produced in TOU cells, 6XHis-eGFP was not secreted by the hairy roots of this plant species.

Fluorescence measurements of the root culture medium of *Nicotiana tabacum* (Nt) and the three edible species *Brassica rapa* (Ver), *Spinacia oleracea* (MAT) and *Daucus carota* (TOU) also confirm that VER is the most efficient species for the secretion of 6XHis-eGFP (Figure 6).

### D. 6XHis-eGFP is efficiently secreted by the Brassicaceae family

Other species belonging to the Brassicaceae family were tested for the secretion of 6XHis-eGFP.
Secreted 6XHis-eGFP was quantified by fluorometry in the culture medium of 20 day-old independent clones. The identification number of each independent clone is indicated on the abscise axis. *Brassica rapa* (VER), *Raphanus sativus var. niger* (NOI), *Raphanus sativus* (CER) and *Brassica Oleracea L. Convar* (QUI) belong to the Brassicaceae family, while *Nicotiana tabacum* (Nt) belongs to the Solanaceae family. Not represented on this graph are the *Daucus carota* (TOU) clones from the Apiaceae family which do not produce any detectable level of secreted 6XHis-eGFP.

The most efficient secretion was observed with clones derived from the Brassicaceae family.

### Conclusion

In conclusion, these studies demonstrate that is possible to obtain good production and secretion into the extracellular medium of heterologous proteins from hairy roots obtained by transforming plants belonging to the Brassicaceae family.

### REFERENCES:

Becard G, F. J. (1988). Early events of vesicular-arbuscular mycorrhiza formation on Ri T-DNA transformed roots. New Phytol. , pp. 211-218.
Bulgakov VP. (2008). Functions of rol genes in plant secondary metabolism. Biotechnology advances, pp. 318-324.
Daniell H, S. N. (2009). Plant-made vaccine antigens and biopharmaceuticals. Trends Plant Sci. , pp. 669-679.
Medina-Bolívar F, C. C. (2004). Production of recombinant proteins by hairy roots cultured in plastic sleeve bioreactors. Methods Mol Biol. , pp. 351-63.
Scmülling, T., Schell, J., & Spena, A. (1988). Single genes from Agrobacterium rhizogenes influence plant development. EMBO, pp. 2621-2629.
William R Swindell, M. H. (2007). Transcriptional profiling of Arabidopsis heat shock proteins and transcription factors reveals extensive overlap between heat and non-heat stress response pathways. BMC Genomics, p. 125.
Woods RR, G. B. (2008). Hairy-root organ cultures for the production of human acetylcholinesterase. BMC Biotechnol*.*

## Claims

1. A method for secreting a recombinant protein from hairy roots comprising the steps of:
a) transforming a plant with a strain of *Agrobacterium rhizogenes* and/or with a strain of *Agrobacterium tumefaciens* comprising the *rol* genes;
and
b) transforming said plant with a vector containing an expression cassette comprising a signal peptide and a gene encoding said recombinant protein;
wherein said plant belongs to the Brassicaceae family.

2. A method according to claim 1, wherein step a) and step b) are performed simultaneously by transforming said plant belonging to the Brassicaceae family with a strain of *Agrobacterium rhizogenes,* wherein said strain of *Agrobacterium rhizogenes* contains an expression cassette comprising a gene encoding said recombinant protein.

3. A method according to claim 1 or 2, wherein said plant belonging to the Brassicaceae family is selected from the group consisting of *Raphanus sativus, Raphanus sativus var. niger, Brassica oleracea L. convar* and *Brassica rapa.*

4. A method according to any one of claims 1 to 3, wherein said plant belonging to the Brassicaceae family is *Brassica rapa.*

5. A method according to any one of claims 1 to 4, wherein said strain of *Agrobacterium rhizogenes* is the strain ATCC 25818.

6. A method according to any one of claims 1 to 5, wherein said expression cassette comprises a promoter, a signal peptide, a gene encoding said recombinant protein and a polyadenylation sequence.

7. A method according to claim 6, wherein said promoter is a promoter derived from a virus infecting Brassicaceae plants.

8. A method according to claim 7, wherein said promoter is the cauliflower mosaic virus 35S (CaMV35S) promoter.

9. A method according to any one of claims 6 to 8, wherein said signal peptide is derived from a Brassicaceae plant.

10. A method according to claim 9, wherein said signal peptide is a signal peptide from the Arabidopsis pectin methylesterase AT1G69940 signal peptide or a variant thereof, such as the signal peptide as set forth in SEQ ID NO: 1.

11. A method according to any one of the preceding claims, wherein said recombinant protein is selected from the group consisting of allergens, vaccines, enzymes, enzyme inhibitors, antibodies, antibody fragments, antigens, toxins, anti-microbial peptides, hormones, growth factors, blood proteins (such as albumin, coagulation factors, transferrin), receptors and signaling proteins, protein component of biomedical standards, protein component of cell culture media, fusion or tagged proteins, cystein (disulfide bridges)-rich peptides and proteins, and plant proteins (such as lectins, papain..).

## Patentansprüche

1. Verfahren zum Sezernieren eines rekombinanten Proteins aus Haarwurzeln (hairy roots), das die Schritte umfasst:
a) Transformieren einer Pflanze mit einem Stamm von Agrobacterium rhizogenes und/oder mit einem Stamm von Agrobacterium tumefaciens, der die rol-Gene umfasst;
sowie
b) Transformieren der Pflanze mit einem Vektor, der eine Expressionskassette enthält, die ein Signalpeptid und ein Gen, das das rekombinante Protein kodiert, umfasst;
wobei die Pflanze der Familie der Brassicaceae angehört.

2. Verfahren gemäß Anspruch 1, wobei Schritt a) und Schritt b) gleichzeitig durchgeführt werden, indem die Pflanze, die der Familie der Brassicaceae angehört, mit einem Stamm von Agrobacterium rhizogenes transformiert wird, wobei der Stamm von Agrobacterium rhizogenes eine Expressionskassette enthält, die ein Gen umfasst, das das rekombinante Protein kodiert.

3. Verfahren gemäß Anspruch 1 oder 2, wobei die Pflanze, die der Familie der Brassicaceae angehört, aus der Gruppe bestehend aus Raphanus sativus, Raphanus sativus var. niger, Brassica oleracea L. convar und Brassica rapa ausgewählt wird.

4. Verfahren gemäß einem der Ansprüche 1 bis 3, wobei die Pflanze, die der Brassicaceae-Familie angehört, Brassica rapa ist.

5. Verfahren gemäß einem der Ansprüche 1 bis 4, wobei der Agrobacterium rhizogenes-Stamm der Stamm ATCC 25818 ist.

6. Verfahren gemäß einem der Ansprüche 1 bis 5, wobei die Expressionskassette einen Promotor, ein Signalpeptid, ein Gen, das das rekombinante Protein kodiert, und eine Polyadenylierungssequenz umfasst.

7. Verfahren gemäß Anspruch 6, wobei der Promotor ein Promotor ist, der von einem Virus abgeleitet ist, das Brassicaceae-Pflanzen infiziert.

8. Verfahren gemäß Anspruch 7, wobei der Promotor der Blumenkohl-Mosaikvirus 35S (CaMV35S)-Promotor ist.

9. Verfahren gemäß einem der Ansprüche 6 bis 8, wobei das Signalpeptid von einer Brassicaceae-Pflanze abgeleitet ist.

10. Verfahren gemäß Anspruch 9, wobei das Signalpeptid ein Signalpeptid vom Arabidopsis-Pektinmethylesterase AT1G69940-Signalpeptid oder eine Variante davon, wie das in SEQ ID NO: 1 dargestellte Signalpeptid, ist.

11. Verfahren gemäß einem der vorhergenenden Ansprüche, wobei das rekombinante Protein aus der Gruppe bestehend aus Allergenen, Impfstoffen, Enzymen, Enzyminhibitoren, Antikörpern, Antikörperfragmenten, Antigenen, Toxinen, antimikrobiellen Peptiden, Hormonen, Wachstumsfaktoren, Blutproteinen (wie Albumin, Gerinnungsfaktoren, Transferrin), Rezeptoren und Signalproteinen, Proteinbestandteilen von biomedizinischen Standards, Proteinbestandteil von Zellkulturmedien, Fusionsproteinen oder markierten Proteinen, Cystein-(Disulfidbrücken)-reichen Peptiden und Proteinen sowie Pflanzenproteinen (wie Lektinen, Papain...) ausgewählt wird.

## Revendications

1. Procédé de sécrétion d'une protéine recombinante à partir de racines chevelues, comprenant les étapes suivantes :
a) la transformation d'une plante avec une souche *d'Agrobacterium rhizogenes* et/ou avec une souche *d'Agrobacterium tumefaciens* comprenant les gènes *rol ;*
et
b) la transformation de ladite plante avec un vecteur contenant une cassette d'expression comprenant un peptide signal et un gène codant ladite protéine recombinante ;
dans lequel ladite plante appartient à la famille des brassicacées.

2. Procédé selon la revendication 1, dans lequel l'étape a) et l'étape b) sont réalisées simultanément par la transformation de ladite plante appartenant à la famille des brassicacées avec une souche *d'Agrobacterium rhizogenes,* dans lequel ladite souche *d'Agrobacterium rhizogenes* contient une cassette d'expression comprenant un gène codant ladite protéine recombinante.

3. Procédé selon la revendication 1 ou 2, dans lequel ladite plante appartenant à la famille des brassicacées est choisie dans le groupe constitué de *Raphanus sativus, Raphanus sativus var. niger, Brassica oleracea L. convar* et *Brassica rapa.*

4. Procédé selon l'une quelconque des revendications 1 à 3, dans lequel ladite plante appartenant à la famille des brassicacées est *Brassica rapa.*

5. Procédé selon l'une quelconque des revendications 1 à 4, dans lequel ladite souche d'*Agrobacterium rhizogenes* est la souche ATCC 25818.

6. Procédé selon l'une quelconque des revendications 1 à 5, dans lequel ladite cassette d'expression comprend un promoteur, un peptide signal, un gène codant ladite protéine recombinante et une séquence de polyadénylation.

7. Procédé selon la revendication 6, dans lequel ledit promoteur est un promoteur dérivé d'un virus infectant les plantes brassicacées.

8. Procédé selon la revendication 7, dans lequel ledit promoteur est le promoteur 35S du virus de la mosaïque du chou-fleur (CaMV35S).

9. Procédé selon l'une quelconque des revendications 6 à 8, dans lequel ledit peptide signal est dérivé d'une plante brassicacée.

10. Procédé selon la revendication 9, dans lequel ledit peptide signal est un peptide signal provenant du peptide signal d'AT1G69940 de la pectine méthylestérase d'*Arabidopsis* ou un variant de celui-ci, tel que le peptide signal représenté par SEQ ID NO : 1.

11. Procédé selon l'une quelconque des revendications précédentes, dans lequel ladite protéine recombinante est choisie dans le groupe constitué des allergènes, des vaccins, des enzymes, des inhibiteurs d'enzyme, des anticorps, des fragments d'anticorps, des antigènes, des toxines, des peptides antimicrobiens, des hormones, des facteurs de croissance, des protéines sanguines (telles que l'albumine, les facteurs de coagulation, la transferrine), des récepteurs et des protéines de signalisation, d'un composant protéique d'étalons biomédicaux, d'un composant protéique de milieu de culture cellulaire, des protéines de fusion ou marquées, des peptides et protéines riches en cystéine (ponts disulfure), et des protéines végétales (telles que les lectines, la papaïne...).


### SEQUENCE LISTING

<110> Université de Picardie Jules Vernes
<120> Method for producing recombinant proteins from plant hairy roots
<130> BEP100292
<160> 2
<170> PatentIn version 3.3
<210> 1
   <211> 13583
   <212> DNA
   <213> artificial
<220>
   <223> pRD400-GFP
<400> 1
   ccgggctggt tgccctcgcc gctgggctgg cggccgtcta tggccctgca aacgcgccag     60
   aaacgccgtc gaagccgtgt gcgagacacc gcggccgccg gcgttgtgga tacctcgcgg    120
   aaaacttggc cctcactgac agatgagggg cggacgttga cacttgaggg gccgactcac    180
   ccggcgcggc gttgacagat gaggggcagg ctcgatttcg gccggcgacg tggagctggc    240
   cagcctcgca aatcggcgaa aacgcctgat tttacgcgag tttcccacag atgatgtgga    300
   caagcctggg gataagtgcc ctgcggtatt gacacttgag gggcgcgact actgacagat     360
   gaggggcgcg atccttgaca cttgaggggc agagtgctga cagatgaggg gcgcacctat    420
   tgacatttga ggggctgtcc acaggcagaa aatccagcat ttgcaagggt ttccgcccgt    480
   ttttcggcca ccgctaacct gtcttttaac ctgcttttaa accaatattt ataaaccttg    540
   tttttaacca gggctgcgcc ctgtgcgcgt gaccgcgcac gccgaagggg ggtgcccccc    600
   cttctcgaac cctcccggcc cgctaacgcg ggcctcccat ccccccaggg gctgcgcccc    660
   tcggccgcga acggcctcac cccaaaaatg gcagcgctgg cagtccttgc cattgccggg    720
   atcggggcag taacgggatg ggcgatcagc ccgagcgcga cgcccggaag cattgacgtg    780
   ccgcaggtgc tggcatcgac attcagcgac caggtgccgg gcagtgaggg cggcggcctg    840
   ggtggcggcc tgcccttcac ttcggccgtc ggggcattca cggacttcat ggcggggccg    900
   gcaattttta ccttgggcat tcttggcata gtggtcgcgg gtgccgtgct cgtgttcggg    960
   ggtgcgataa acccagcgaa ccatttgagg tgataggtaa gattataccg aggtatgaaa   1020
   acgagaattg gacctttaca gaattactct atgaagcgcc atatttaaaa agctaccaag   1080
   acgaagagga tgaagaggat gaggaggcag attgccttga atatattgac aatactgata   1140
   agataatata tcttttatat agaagatatc gccgtatgta aggatttcag ggggcaaggc    1200
   ataggcagcg cgcttatcaa tatatctata gaatgggcaa agcataaaaa cttgcatgga   1260
   ctaatgcttg aaacccagga caataacctt atagcttgta aattctatca taattgggta   1320
   atgactccaa cttattgata gtgttttatg ttcagataat gcccgatgac tttgtcatgc   1380
   agctccaccg attttgagaa cgacagcgac ttccgtccca gccgtgccag gtgctgcctc   1440
   agattcaggt tatgccgctc aattcgctgc gtatatcgct tgctgattac gtgcagcttt 1500
   cccttcaggc gggattcata cagcggccag ccatccgtca tccatatcac cacgtcaaag 1560
   ggtgacagca ggctcataag acgccccagc gtcgccatag tgcgttcacc gaatacgtgc 1620
   gcaacaaccg tcttccggag actgtcatac gcgtaaaaca gccagcgctg gcgcgattta 1680
   gccccgacat agccccactg ttcgtccatt tccgcgcaga cgatgacgtc actgcccggc 1740
   tgtatgcgcg aggttaccga ctgcggcctg agttttttaa gtgacgtaaa atcgtgttga 1800
   ggccaacgcc cataatgcgg gctgttgccc ggcatccaac gccattcatg gccatatcaa 1860
   tgattttctg gtgcgtaccg ggttgagaag cggtgtaagt gaactgcagt tgccatgttt 1920
   tacggcagtg agagcagaga tagcgctgat gtccggcggt gcttttgccg ttacgcacca 1980
   ccccgtcagt agctgaacag gagggacagc tgatagacac agaagccact ggagcacctc 2040
   aaaaacacca tcatacacta aatcagtaag ttggcagcat cacccataat tgtggtttca 2100
   aaatcggctc cgtcgatact atgttatacg ccaactttga aaacaacttt gaaaaagctg 2160
   ttttctggta tttaaggttt tagaatgcaa ggaacagtga attggagttc gtcttgttat 2220
   aattagcttc ttggggtatc tttaaatact gtagaaaaga ggaaggaaat aataaatggc 2280
   taaaatgaga atatcaccgg aattgaaaaa actgatcgaa aaataccgct gcgtaaaaga 2340
   tacggaagga atgtctcctg ctaaggtata taagctggtg ggagaaaatg aaaacctata 2400
   tttaaaaatg acggacagcc ggtataaagg gaccacctat gatgtggaac gggaaaagga 2460
   catgatgcta tggctggaag gaaagctgcc tgttccaaag gtcctgcact ttgaacggca 2520
   tgatggctgg agcaatctgc tcatgagtga ggccgatggc gtcctttgct cggaagagta 2580
   tgaagatgaa caaagccctg aaaagattat cgagctgtat gcggagtgca tcaggctctt 2640
   tcactccatc gacatatcgg attgtcccta tacgaatagc ttagacagcc gcttagccga 2700
   attggattac ttactgaata acgatctggc cgatgtggat tgcgaaaact gggaagaaga 2760
   cactccattt aaagatccgc gcgagctgta tgatttttta aagacggaaa agcccgaaga 2820
   ggaacttgtc ttttcccacg gcgacctggg agacagcaac atctttgtga aagatggcaa 2880
   agtaagtggc tttattgatc ttgggagaag cggcagggcg gacaagtggt atgacattgc 2940
   cttctgcgtc cggtcgatca gggaggatat cggggaagaa cagtatgtcg agctattttt 3000
   tgacttactg gggatcaagc ctgattggga gaaaataaaa tattatattt tactggatga 3060
   attgttttag tacctagatg tggcgcaacg atgccggcga caagcaggag cgcaccgact 3120
   tcttccgcat caagtgtttt ggctctcagg ccgaggccca cggcaagtat ttgggcaagg 3180
   ggtcgctggt attcgtgcag ggcaagattc ggaataccaa gtacgagaag gacggccaga 3240
   cggtctacgg gaccgacttc attgccgata aggtggatta tctggacacc aaggcaccag 3300
   gcgggtcaaa tcaggaataa gggcacattg ccccggcgtg agtcggggca atcccgcaag 3360
   gagggtgaat gaatcggacg tttgaccgga aggcatacag gcaagaactg atcgacgcgg 3420
   ggttttccgc cgaggatgcc gaaaccatcg caagccgcac cgtcatgcgt gcgccccgcg 3480
   aaaccttcca gtccgtcggc tcgatggtcc agcaagctac ggccaagatc gagcgcgaca 3540
   gcgtgcaact ggctccccct gccctgcccg cgccatcggc cgccgtggag cgttcgcgtc 3600
   gtctcgaaca ggaggcggca ggtttggcga agtcgatgac catcgacacg cgaggaacta 3660
   tgacgaccaa gaagcgaaaa accgccggcg aggacctggc aaaacaggtc agcgaggcca 3720
   agcaggccgc gttgctgaaa cacacgaagc agcagatcaa ggaaatgcag ctttccttgt 3780
   tcgatattgc gccgtggccg gacacgatgc gagcgatgcc aaacgacacg gcccgctctg 3840
   ccctgttcac cacgcgcaac aagaaaatcc cgcgcgaggc gctgcaaaac aaggtcattt 3900
   tccacgtcaa caaggacgtg aagatcacct acaccggcgt cgagctgcgg gccgacgatg 3960
   acgaactggt gtggcagcag gtgttggagt acgcgaagcg cacccctatc ggcgagccga 4020
   tcaccttcac gttctacgag ctttgccagg acctgggctg gtcgatcaat ggccggtatt 4080
   acacgaaggc cgaggaatgc ctgtcgcgcc tacaggcgac ggcgatgggc ttcacgtccg 4140
   accgcgttgg gcacctggaa tcggtgtcgc tgctgcaccg cttccgcgtc ctggaccgtg 4200
   gcaagaaaac gtcccgttgc caggtcctga tcgacgagga aatcgtcgtg ctgtttgctg 4260
   gcgaccacta cacgaaattc atatgggaga agtaccgcaa gctgtcgccg acggcccgac 4320
   ggatgttcga ctatttcagc tcgcaccggg agccgtaccc gctcaagctg gaaaccttcc 4380
   gcctcatgtg cggatcggat tccacccgcg tgaagaagtg gcgcgagcag gtcggcgaag 4440
   cctgcgaaga gttgcgaggc agcggcctgg tggaacacgc ctgggtcaat gatgacctgg 4500
   tgcattgcaa acgctagggc cttgtggggt cagttccggc tgggggttca gcagccagcg 4560
   ctttactggc atttcaggaa caagcgggca ctgctcgacg cacttgcttc gctcagtatc 4620
   gctcgggacg cacggcgcgc tctacgaact gccgataaac agaggattaa aattgacaat 4680
   tgtgattaag gctcagattc gacggcttgg agcggccgac gtgcaggatt tccgcgagat 4740
   ccgattgtcg gccctgaaga aagctccaga gatgttcggg tccgtttacg agcacgagga 4800
   gaaaaagccc atggaggcgt tcgctgaacg gttgcgagat gccgtggcat tcggcgccta 4860
   catcgacggc gagatcattg ggctgtcggt cttcaaacag gaggacggcc ccaaggacgc 4920
   tcacaaggcg catctgtccg gcgttttcgt ggagcccgaa cagcgaggcc gaggggtcgc 4980
   cggtatgctg ctgcgggcgt tgccggcggg tttattgctc gtgatgatcg tccgacagat 5040
   tccaacggga atctggtgga tgcgcatctt catcctcggc gcacttaata tttcgctatt 5100
   ctggagcttg ttgtttattt cggtctaccg cctgccgggc ggggtcgcgg cgacggtagg 5160
   cgctgtgcag ccgctgatgg tcgtgttcat ctctgccgct ctgctaggta gcccgatacg 5220
   attgatggcg gtcctggggg ctatttgcgg aactgcgggc gtggcgctgt tggtgttgac 5280
   accaaacgca gcgctagatc ctgtcggcgt cgcagcgggc ctggcggggg cggtttccat 5340
   ggcgttcgga accgtgctga cccgcaagtg gcaacctccc gtgcctctgc tcacctttac 5400
   cgcctggcaa ctggcggccg gaggacttct gctcgttcca gtagctttag tgtttgatcc 5460
   gccaatcccg atgcctacag gaaccaatgt tctcggcctg gcgtggctcg gcctgatcgg 5520
   agcgggttta acctacttcc tttggttccg ggggatctcg cgactcgaac ctacagttgt 5580
   ttccttactg ggctttctca gccccagatc tggggtcgat cagccgggga tgcatcaggc 5640
   cgacagtcgg aacttcgggt ccccgacctg taccattcgg tgagcaatgg ataggggagt 5700
   tgatatcgtc aacgttcact tctaaagaaa tagcgccact cagcttcctc agcggcttta 5760
   tccagcgatt tcctattatg tcggcatagt tctcaagatc gacagcctgt cacggttaag 5820
   cgagaaatga ataagaaggc tgataattcg gatctctgcg agggagatga tatttgatca 5880
   caggcagcaa cgctctgtca tcgttacaat caacatgcta ccctccgcga gatcatccgt 5940
   gtttcaaacc cggcagctta gttgccgttc ttccgaatag catcggtaac atgagcaaag 6000
   tctgccgcct tacaacggct ctcccgctga cgccgtcccg gactgatggg ctgcctgtat 6060
   cgagtggtga ttttgtgccg agctgccggt cggggagctg ttggctggct ggtggcagga 6120
   tatattgtgg tgtaaacaaa ttgacgctta gacaacttaa taacacattg cggacgtttt 6180
   taatgtactg gggtggtttt tcttttcacc agtgagacgg gcaacagctg attgcccttc 6240
   accgcctggc cctgagagag ttgcagcaag cggtccacgc tggtttgccc cagcaggcga 6300
   aaatcctgtt tgatggtggt tccgaaatcg gcaaaatccc ttataaatca aaagaatagc 6360
   ccgagatagg gttgagtgtt gttccagttt ggaacaagag tccactatta aagaacgtgg 6420
   actccaacgt caaagggcga aaaaccgtct atcagggcga tggcccacta cgtgaaccat 6480
   cacccaaatc aagttttttg gggtcgaggt gccgtaaagc actaaatcgg aaccctaaag 6540
   ggagcccccg atttagagct tgacggggaa agccggcgaa cgtggcgaga aaggaaggga 6600
   agaaagcgaa aggagcgggc gccattcagg ctgcgcaact gttgggaagg gcgatcggtg 6660
   cgggcctctt cgctattacg ccagctggcg aaagggggat gtgctgcaag gcgattaagt 6720
   tgggtaacgc cagggttttc ccagtcacga cgttgtaaaa cgacggccag tgaattcgag 6780
   ctcggtaccc ctactccaaa aatgtcaaag atacagtctc agaagaccaa agggctattg 6840
   agacttttca acaaagggta atttcgggaa acctcctcgg attccattgc ccagctatct 6900
   gtcacttcat cgaaaggaca gtagaaaagg aaggtggctc ctacaaatgc catcattgcg 6960
   ataaaggaaa ggctatcatt caagatgcct ctgccgacag tggtcccaaa gatggacccc 7020
   cacccacgag gagcatcgtg gaaaaagaag acgttccaac cacgtcttca aagcaagtgg 7080
   attgatgtga catctccact gacgtaaggg atgacgcaca atcccacccc tactccaaaa 7140
   atgtcaaaga tacagtctca gaagaccaaa gggctattga gacttttcaa caaagggtaa 7200
   tttcgggaaa cctcctcgga ttccattgcc cagctatctg tcacttcatc gaaaggacag 7260
   tagaaaagga aggtggctcc tacaaatgcc atcattgcga taaaggaaag gctatcattc 7320
   aagatgcctc tgccgacagt ggtcccaaag atggaccccc acccacgagg agcatcgtgg 7380
   aaaaagaaga cgttccaacc acgtcttcaa agcaagtgga ttgatgtgac atctccactg 7440
   acgtaaggga tgacgcacaa tcccactatc cttcgcaaga cccttcctct atataaggaa 7500
   gttcatttca tttggagagg acagcccaag cttaaaacaa caatgggata cacaaatgtg 7560
   tccattttat taggcctgtt gatggtcttt gttacaccga tggtgttcgc agatcccatg 7620
   catcatcatc atcaccaccc catggtgagc aagggcgagg agctgttcac cggggtggtg 7680
   cccatcctgg tcgagctgga cggcgacgta aacggccaca agttcagcgt gtccggcgag 7740
   ggcgagggcg atgccaccta cggcaagctg accctgaagt tcatctgcac caccggcaag 7800
   ctgcccgtgc cctggcccac cctcgtgacc accctgacct acggcgtgca gtgcttcagc 7860
   cgctaccccg accacatgaa gcagcacgac ttcttcaagt ccgccatgcc cgaaggctac 7920
   gtccaggagc gcaccatctt cttcaaggac gacggcaact acaagacccg cgccgaggtg 7980
   aagttcgagg gcgacaccct ggtgaaccgc atcgagctga agggcatcga cttcaaggag 8040
   gacggcaaca tcctggggca caagctggag tacaactaca acagccacaa cgtctatatc 8100
   atggccgaca agcagaagaa cggcatcaag gtgaacttca agatccgcca caacatcgag 8160
   gacggcagcg tgcagctcgc cgaccactac cagcagaaca cccccatcgg cgacggcccc 8220
   gtgctgctgc ccgacaacca ctacctgagc acccagtccg ccctgagcaa agaccccaac 8280
   gagaagcgcg atcacatggt cctgctggag ttcgtgaccg ccgccgggat cactctcggc 8340
   atggacgagc tgtacaagta aagcggccgc gactctagaa ttcgctgaaa tcaccagtct 8400
   ctctctacaa atctatctct ctctattttc tccataaata atgtgtgagt agtttcccga 8460
   taagggaaat tagggttctt atagggtttc gctcatgtgt tgagcatata agaaaccctt 8520
   agtatgtatt tgtatttgta aaatacttct atcaataaaa tttctaattc ctaaaaccaa 8580
   aatccagtac taaaatccag atcctctaga gtcgacctgc aggcatgcaa gcttggcgta 8640
   atcatggtca tagctgtttc ctgtgtgaaa ttgttatccg ctcacaattc cacacaacat 8700
   acgagccgga agcataaagt gtaaagcctg gggtgcctaa tgagtgagct aactcacatt 8760
   aattgcgttg cgctcactgc ccgctttcca gtcgggaaac ctgtcgtgcc agctgcatta 8820
   atgaatcggc caacgcgcgg ggagaggcgg tttgcgtatt gggccaaaga caaaagggcg 8880
   acattcaacc gattgaggga gggaaggtaa atattgacgg aaattattca ttaaaggtga 8940
   attatcaccg tcaccgactt gagccatttg ggaattagag ccagcaaaat caccagtagc 9000
   accattacca ttagcaaggc cggaaacgtc accaatgaaa ccatcgatag cagcaccgta 9060
   atcagtagcg acagaatcaa gtttgccttt agcgtcagac tgtagcgcgt tttcatcggc 9120
   attttcggtc atagccccct tattagcgtt tgccatcttt tcataatcaa aatcaccgga 9180
   accagagcca ccaccggaac cgcctccctc agagccgcca ccctcagaac cgccaccctc 9240
   agagccacca ccctcagagc cgccaccaga accaccacca gagccgccgc cagcattgac 9300
   aggaggcccg atctagtaac atagatgaca ccgcgcgcga taatttatcc tagtttgcgc 9360
   gctatatttt gttttctatc gcgtattaaa tgtataattg cgggactcta atcataaaaa 9420
   cccatctcat aaataacgtc atgcattaca tgttaattat tacatgctta acgtaattca 9480
   acagaaatta tatgataatc atcgcaagac cggcaacagg attcaatctt aagaaacttt 9540
   attgccaaat gtttgaacga tcggggatca tccgggtctg tggcgggaac tccacgaaaa 9600
   tatccgaacg cagcaagata tcgcggtgca tctcggtctt gcctgggcag tcgccgccga 9660
   cgccgttgat gtggacgccg ggcccgatca tattgtcgct caggatcgtg gcgttgtgct 9720
   tgtcggccgt tgctgtcgta atgatatcgg caccttcgac cgcctgttcc gcagagatcc 9780
   cgtgggcgaa gaactccagc atgagatccc cgcgctggag gatcatccag ccggcgtccc 9840
   ggaaaacgat tccgaagccc aacctttcat agaaggcggc ggtggaatcg aaatctcgtg 9900
   atggcaggtt gggcgtcgct tggtcggtca tttcgaaccc cagagtcccg ctcagaagaa 9960
   ctcgtcaaga aggcgataga aggcgatgcg ctgcgaatcg ggagcggcga taccgtaaag 10020
   cacgaggaag cggtcagccc attcgccgcc aagctcttca gcaatatcac gggtagccaa 10080
   cgctatgtcc tgatagcggt ccgccacacc cagccggcca cagtcgatga atccagaaaa 10140
   gcggccattt tccaccatga tattcggcaa gcaggcatcg ccatgggtca cgacgagatc 10200
   ctcgccgtcg ggcatgcgcg ccttgagcct ggcgaacagt tcggctggcg cgagcccctg 10260
   atgctcttcg tccagatcat cctgatcgac aagaccggct tccatccgag tacgtgctcg 10320
   ctcgatgcga tgtttcgctt ggtggtcgaa tgggcaggta gccggatcaa gcgtatgcag 10380
   ccgccgcatt gcatcagcca tgatggatac tttctcggca ggagcaaggt gagatgacag 10440
   gagatcctgc cccggcactt cgcccaatag cagccagtcc cttcccgctt cagtgacaac 10500
   gtcgagcaca gctgcgcaag gaacgcccgt cgtggccagc cacgatagcc gcgctgcctc 10560
   gtcctgcagt tcattcaggg caccggacag gtcggtcttg acaaaaagaa ccgggcgccc 10620
   ctgcgctgac agccggaaca cggcggcatc agagcagccg attgtctgtt gtgcccagtc 10680
   atagccgaat agcctctcca cccaagcggc cggagaacct gcgtgcaatc catcttgttc 10740
   aatcatgcga aacgatccag atccggtgca gattatttgg attgagagtg aatatgagac 10800
   tctaattgga taccgagggg aatttatgga acgtcagtgg agcatttttg acaagaaata 10860
   tttgctagct gatagtgacc ttaggcgact tttgaacgcg caataatggt ttctgacgta 10920
   tgtgcttagc tcattaaact ccagaaaccc gcggctgagt ggctccttca acgttgcggt 10980
   tctgtcagtt ccaaacgtaa aacggcttgt cccgcgtcat cggcgggggt cataacgtga 11040
   ctcccttaat tctccgctca tgatcagatt gtcgtttccc gccttcagtt taaactatca 11100
   gtgtttgaca ggatatattg gcgggtaaac ctaagagaaa agagcgttta ttagaataat 11160
   cggatattta aaagggcgtg aaaaggttta tccgttcgtc catttgtatg tgcatgccaa 11220
   ccacagggtt ccccagatct ggcgccggcc agcgagacga gcaagattgg ccgccgcccg 11280
   aaacgatccg acagcgcgcc cagcacaggt gcgcaggcaa attgcaccaa cgcatacagc 11340
   gccagcagaa tgccatagtg ggcggtgacg tcgttcgagt gaaccagatc gcgcaggagg 11400
   cccggcagca ccggcataat caggccgatg ccgacagcgt cgagcgcgac agtgctcaga 11460
   attacgatca ggggtatgtt gggtttcacg tctggcctcc ggaccagcct ccgctggtcc 11520
   gattgaacgc gcggattctt tatcactgat aagttggtgg acatattatg tttatcagtg 11580
   ataaagtgtc aagcatgaca aagttgcagc cgaatacagt gatccgtgcc gccctggacc 11640
   tgttgaacga ggtcggcgta gacggtctga cgacacgcaa actggcggaa cggttggggg  11700
   ttcagcagcc ggcgctttac tggcacttca ggaacaagcg ggcgctgctc gacgcactgg  11760
   ccgaagccat gctggcggag aatcatacgc attcggtgcc gagagccgac gacgactggc  11820
   gctcatttct gatcgggaat gcccgcagct tcaggcaggc gctgctcgcc taccgcgatg  11880
   gcgcgcgcat ccatgccggc acgcgaccgg gcgcaccgca gatggaaacg gccgacgcgc  11940
   agcttcgctt cctctgcgag gcgggttttt cggccgggga cgccgtcaat gcgctgatga  12000
   caatcagcta cttcactgtt ggggccgtgc ttgaggagca ggccggcgac agcgatgccg  12060
   gcgagcgcgg cggcaccgtt gaacaggctc cgctctcgcc gctgttgcgg gccgcgatag  12120
   acgccttcga cgaagccggt ccggacgcag cgttcgagca gggactcgcg gtgattgtcg  12180
   atggattggc gaaaaggagg ctcgttgtca ggaacgttga aggaccgaga aagggtgacg  12240
   attgatcagg accgctgccg gagcgcaacc cactcactac agcagagcca tgtagacaac  12300
   atcccctccc cctttccacc gcgtcagacg cccgtagcag cccgctacgg gctttttcat  12360
   gccctgccct agcgtccaag cctcacggcc gcgctcggcc tctctggcgg ccttctggcg  12420
   ctcttccgct tcctcgctca ctgactcgct gcgctcggtc gttcggctgc ggcgagcggt  12480
   atcagctcac tcaaaggcgg taatacggtt atccacagaa tcaggggata acgcaggaaa  12540
   gaacatgtga gcaaaaggcc agcaaaaggc caggaaccgt aaaaaggccg cgttgctggc  12600
   gtttttccat aggctccgcc cccctgacga gcatcacaaa aatcgacgct caagtcagag  12660
   gtggcgaaac ccgacaggac tataaagata ccaggcgttt ccccctggaa gctccctcgt  12720
   gcgctctcct gttccgaccc tgccgcttac cggatacctg tccgcctttc tcccttcggg  12780
   aagcgtggcg cttttccgct gcataaccct gcttcggggt cattatagcg attttttcgg  12840
   tatatccatc ctttttcgca cgatatacag gattttgcca aagggttcgt gtagactttc  12900
   cttggtgtat ccaacggcgt cagccgggca ggataggtga agtaggccca cccgcgagcg  12960
   ggtgttcctt cttcactgtc ccttattcgc acctggcggt gctcaacggg aatcctgctc  13020
   tgcgaggctg gccggctacc gccggcgtaa cagatgaggg caagcggatg gctgatgaaa  13080
   ccaagccaac caggaagggc agcccaccta tcaaggtgta ctgccttcca gacgaacgaa  13140
   gagcgattga ggaaaaggcg gcggcggccg gcatgagcct gtcggcctac ctgctggccg  13200
   tcggccaggg ctacaaaatc acgggcgtcg tggactatga gcacgtccgc gagctggccc  13260
   gcatcaatgg cgacctgggc cgcctgggcg gcctgctgaa actctggctc accgacgacc  13320
   cgcgcacggc gcggttcggt gatgccacga tcctcgccct gctggcgaag atcgaagaga  13380
   agcaggacga gcttggcaag gtcatgatgg gcgtggtccg cccgagggca gagccatgac  13440
   ttttttagcc gctaaaacgg ccggggggtg cgcgtgattg ccaagcacgt ccccatgcgc  13500
   tccatcaaga agagcgactt cgcggagctg gtgaagtaca tcaccgacga gcaaggcaag  13560
   accgagcgcc tttgcgacgc tca                                          13583
<210> 2
   <211> 25
   <212> PRT
   <213> Arabidopsis thaliana
<400> 2
   Met Gly Tyr Thr Asn Val Ser Ile Leu Leu Gly Leu Leu Met Val Phe
   1               5                   10                  15
   Val Thr Pro Met Val Phe Ala Asp Pro
               20                  25